# EUROPEAN PATENT SPECIFICATION

(11) **EP 1 889 592 B1**
(45) Date of publication and mention of the grant of the patent: **10.01.2018**
(21) Application number: 06732262.8
(22) Date of filing: 24.04.2006
(51) Int. Cl.: A61H 3/00

(54) **THIGH PART MOUNTING DEVICE FOR WALKING ASSISTING DEVICE**
VORRICHTUNG ZUR BEFESTIGUNG EINES OBERSCHENKELTEILS FÜR EINE GEHHILFEVORRICHTUNG
DISPOSITIF DE MONTAGE DE PARTIE CUISSE POUR DISPOSITIF D'AIDE A LA MARCHE

(30) Priority: 17.05.2005 JP 2005143423
(43) Date of publication of application: 20.02.2008
(73) Proprietor: HONDA MOTOR CO., LTD., Tokyo 107-8556 (JP)
(72) Inventor: HIRATA, Takashi, Wako-shi, Saitama 351-0193 (JP); SHIMADA, Kei, Wako-shi, Saitama 351-0193 (JP); FUJII, Takako, Kyoto-shi Kyoto 601-8530 (JP)
(74) Representative: Hague, Alison Jane
(86) International application number: PCT/JP2006/308551
(87) International publication number: WO 2006/123515

(56) References cited:
- WO-A1-03/091014
- JP-A- 2002 301 124
- JP-A- 2003 220 102
- JP-A- 2003 220 102
- JP-C1- 6 380
- US-A1- 2003 223 844

## Description

### TECHNICAL FIELD

The present application claims priority from Japanese application No. 2005-143423 filed May 17, 2005.

The present invention relates to a femoral support member for a walking assistance device, and in particular to a femoral support member for a walking assistance device in which the femoral support member secures a free end of an assist force transmitting arm of a power actuator for assisting the muscular force of a wearer having a deficiency in the functions of his lower limbs to a femoral region of the wearer.

### BACKGROUND OF THE INVENTION

Various walking assistance devices have been proposed (see Japanese patent laid open publication No. 07-163607, for instance) for the purpose of providing an assistance to a person who has a difficulty in walking by himself owing to the lack of muscular force, and such a device typically includes a power actuator such as an electric motor fitted on the wearer near his hip joint or knee joint to assist the movement of his lower limbs.

In the walking assistance device disclosed in Japanese patent laid open publication No. 07-163607, a femoral support member is worn around a femoral region of the wearer, and an actuating force of a power actuator is transmitted to the femoral region of the wearer via the femoral support member. The femoral support member is provided with a cylindrical shape so as to surround the femoral region and be firmly secured thereto, and is made of soft and flexible material such as elastic fabric, leather and plastic sheet. The femoral support member is configured to be opened at a part thereof so as to be detachably fitted around the femoral region.

### BRIEF SUMMARY OF THE INVENTION

### TASK TO BE ACCOMPLISHES BY THE INVENTION

However, the femoral support member disclosed in Japanese patent laid open publication No. 07-163607 is configured so as to substantially entirely wrap around the femoral region. The femoral region includes particularly thick muscles as compared with muscles of other body parts, and experiences a greatest change in the cross section (circumferential length) as the muscles extend and contract. Therefore, when the femoral support member is fastened around the femoral region at an appropriate tension when the wearer is standing and the muscles of the femoral region extend, the femoral support member will apply excessive pressure to the femoral region when the wearer squats and the muscles of the femoral region contract. Conversely, if the femoral support member is at a proper tension when the wearer squats, the femoral support member will become loose when the wearer stands.

Japanese patent laid open publication No. 07-163607 suggests the use of highly flexible material, but in practice it is extremely difficult to provide a femoral support member that can transmit the force of a power actuator to a femoral region of the wearer without obstructing muscle movements or causing discomfort or pain to the wearer.

The present invention was made with the aim of eliminating such problems of the prior art, and has a primary object to provide a femoral support member for a walking assistance device that would not obstruct the muscle movement of the wearer or become loose during use.

JP 2003 220102A discloses a walking assistance device having belts for securing an assist force transmitting arm to a femoral region of a wearer.

US 2003/223884 discloses an inflatable annular cushion inserted in a rigid outer ring in a shoulder, elbow or wrist assembly.

### MEANS TO ACCOMPLISH THE TASKS

According to the present invention, such an object can be accomplished by providing a walking assistance device, comprising a hip support member adapted to be secured to a hip of a wearer, a power actuator secured to the hip support member and having a drive shaft, an assist force transmitting arm extending radially from the drive shaft and a femoral support member for securing a free end of the assist force transmitting arm to a femoral region of the wearer, characterized in that: the femoral support member is adapted to engage the femoral region via an air pad including a bag filled with air, the femoral support member further comprises a stay that supports the air pad on a free end of the assist force transmitting arm and is adapted to at least partly surround the femoral region of the wearer, and in that the femoral support member comprises a front support plate attached to the stay so as to oppose a front face of the femoral region and a rear support plate attached to the stay so as to oppose a rear face of the femoral region, the front support plate being adapted to engage a corresponding part of the femoral region via the air pad.

Thus, owing to the air cushioning action of the air pad, the air pad can readily deform itself by a large displacement even when the wearer squats and the femoral region expands.

Since the femoral support member further comprises a stay that supports the pad on a free end of the assist force transmitting arm and at least partly surrounds the femoral region of the wearer, the femoral region can be supported by the free end of the assist force transmitting arm in a stable fashion. In particular if the femoral support member comprises a front support plate attached to the stay so as to oppose a front face of the femoral region and a rear support plate attached to the stay so as to oppose a rear face of the femoral region, the front support plate engaging a corresponding part of the femoral region via the air pad, in at least preferred embodiments the pressure supporting surface area of the femoral region can be maximized. In view of the anatomy of a human body, it is preferable if the front support plate is vertically longer than the rear support plate and/or the rear support plate is wider than the front support plate.

If the assist force transmitting arm consists of a member that is substantially rigid with respect to a rotational direction thereof, the distance between the power actuator secured to a part adjacent to the hip joint and the femoral support member remains fixed, and the femoral support member would not become loose during use.

To favorably accommodate the movement of the femoral region, it is preferable if each support plate is rotatably attached to the stay and/or each support plate is attached to the stay via a spring joint.

In particular, if the support plates are applied to a body part via a pad comprising a bag filled with semi-fluid material such as rolling granules, it can accommodate a shifting of the femoral support member relative to the body of the wearer which is caused by the disagreement between the position at which the power actuator and power transmitting device are secured to a body part and an axis of movement (center of an articulate joint), and the natural movement of the lower limb would not be obstructed.

If the walking assistance device further comprises a pump for supplementing air in the air pad, the air in the air pad may be added or removed at will so that the femoral support member can be worn in a favorable manner.

### BRIEF DESCRIPTION OF THE DRAWINGS

Now the present invention is described in the following with reference to the appended drawings, in which:
Figure 1 is a perspective view showing an overall structure of a walking assistance device embodying the present invention;
Figure 2 is a plan view of the femoral support member;
Figure 3 is a perspective view of the femoral support member; and
Figure 4 is a perspective view of the front support of the femoral support member.

### DETAILED DESCRIPTION OF THE PREFERRED EMBODIMENTS

Now the present invention is described in the following in more detail in terms of a concrete embodiment with reference to the appended drawings.

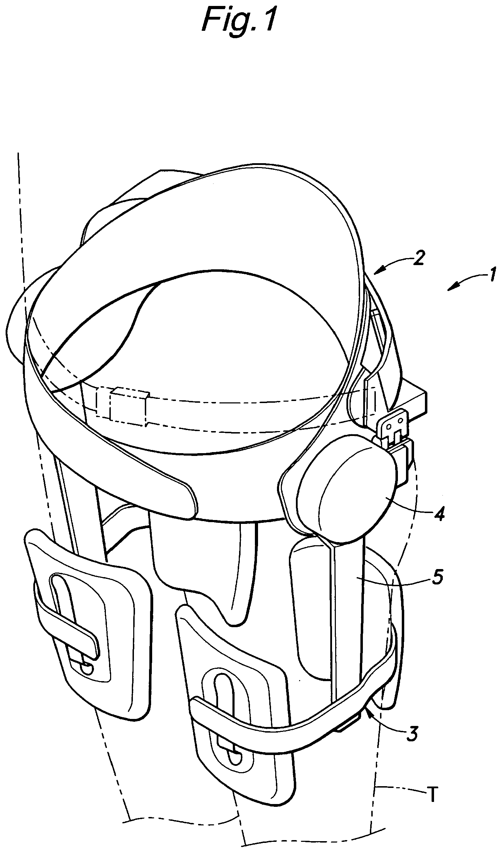

Figure 1 is a simplified structural view of a walking assistance device embodying the present invention. This walking assistance device 1 comprises a hip support member 2, a pair of femoral support members 3 and an actuator 4 attached to the hip support member 2 for producing an assist power for each femoral support members 3. Each actuator 4 is secured to a side of a hip joint of a wearer by securing the hip support member 2 to the hip portion of the wearer. By wearing the femoral support member 3 on a femoral region T of the wearer, the assist force which is applied to a free end of a torque arm 5 extending from an output shaft of the actuator 4 along an outer side of the femoral region T is transmitted to the femoral region T.

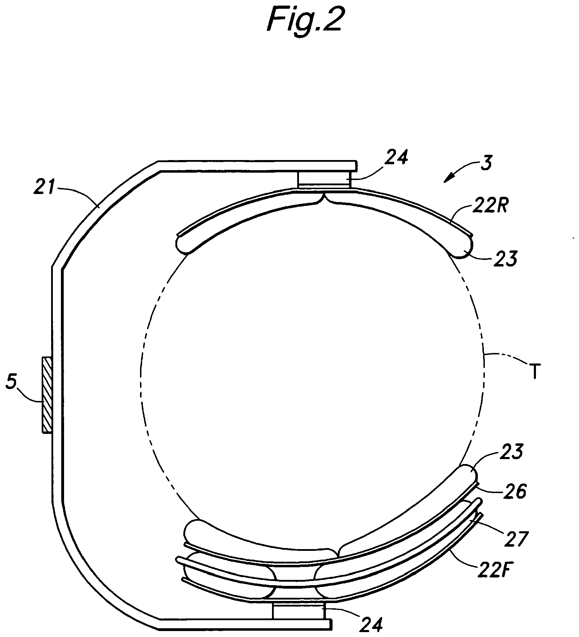

Referring to Figure 2, each femoral support member 3 connected to the free end of the torque arm 5 comprises a stay 21 consisting of a flat bar bent into the shape of letter-U as seen in plan view, a pair of support plates 22F and 22R each made of a thin plate member and attached to the inner face of a corresponding one of the two opposing ends of the stay 21 and a pair of pads 23 attached to the opposing inner surfaces of the two support plates 22F and 22R, respectively, or the surfaces that engage the femoral region T. The stay 21 is substantially rigid with respect to a rotational direction thereof but is somewhat flexible in the inward and outward directions. The outer surface of each support plate 22F and 22R is connected to a flexible connecting plate 24 (that serves the dual purposes of a hinge and a sheet spring) at an upper and a lower part of the connecting plate 24, and the stay 21 is connected to an intermediate point of the flexible connecting plate 24. The stay 21, support plates 22F and 22R and flexible connecting plate 24 are made of plastic material reinforced with fibers such as carbon fibers and Kevler fibers for the purpose of achieving both a high rigidity and a light weight. The joint between each torque arm 5 and the corresponding stay 21 as well as the joint between the stay 21 and each support plate 22F and 22R may comprise a threaded bolt and nut that are used in combination with a plurality of holes for passing the bolt so that the position of the joint may be adjusted to the build of the wearer by selecting one of the holes.

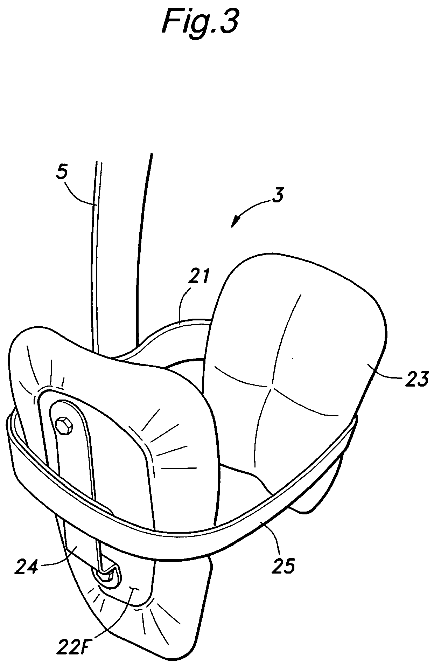

The support plates 22F and 22R are configured so as to interpose the femoral region T between the opposing surfaces thereof via the pads 23 from front and rear. The front support plate 22F engaging the front surface of the femoral region T is vertically more elongated than the rear support plate 22R engaging the rear surface of the femoral region T, and the rear support plate 22R is wider than the front support plate 22F. As shown in Figure 3, the femoral support member 3 is secured to the femoral region T by wrapping a web belt 25 around the femoral region T with the support plates 22F and 22R interposed between them, and joining the two ends of the web belt 25 by using a planar fastener.

The rear support plate 22R engages the hamstring of the wearer that includes a femoral biceps, and the front support plate 22F engages the femoral quadriceps of the wearer. To avoid the rear support plate 22R from interfering with the back of the knee of the wearer when the wearer squats, the rear support plate 22R has a raised lower edge and is shorter by the front support plate F by the corresponding length. Because the muscular force of the femoral quadriceps is about 1.6 times greater than that of the hamstring when supporting the human weight, the efficiency of the force transmission from the torque arm 5 can be increased by increasing the vertical dimension of the front support plate 22F so as to increase the load supporting capability and arranging the central path of force transmission in parallel with the femoral bone.

The pad 23 that engages the femoral region T consists of a bag made by sewing sheet material and filled with foamed plastic granules (beads).

The sheet material for the bag is desired to be pliant and low in friction. In terms of a capability to retain shape, thermoplastic plastic such as nylon, polyester, polypropylene is preferred. In particular, non-woven fabric, knit fabric and woven fabrics made of fibers of such plastic material are preferred. Absorption property is also important in view of the inevitable sweating of the wearer, but as this pad 23 is typically worn over a garment worn by the wearer, the material is required to be highly air permeable at least.

The beads filled in the bag may be readily formed by shaping plastic material into granules while the plastic material is being foamed or by breaking lumps of suitably foamed plastic material into fine particles. The plastic material may consist of those that have a molecular structure free from polar groups and are low in friction, such as polystyrene and polypropylene. If the particle size is too small, comfort to the wearer is impaired because the particles would not readily undergo elastic deformation and the wearer may feel the particles to be too hard. If the particle size is too great, the bag demonstrates an irregular outer contour, and this impairs the appearance of the pad 23. Based on such considerations, the particle size is desired to be in the range of 0.2 mm to 2mm.

If the amount of the beads that fill the bag is too small, the bag is unable to properly conform to the outer surface of the femoral region T. Therefore, the amount of the beads that fill the bag should be such that the beads can freely move inside the bag and a layer of beads having a suitable thickness may be always present between the femoral region T and pad 23 when the pad 23 is pressed against the femoral region T.

Even though the beads are extremely light in weight, the beads tend to sink downward under their own weight when the pad 23 is not worn by the wearer. It is therefore preferable to divide the bag into a number of small cells by sewing so that the beads may be favorably distributed over the entire bag as illustrated in Figure 3. Thereby, when the femoral support member 3 is worn on the femoral region T, the beads that may be settling in a lower part of each cell of the bag may move upward so as to conform to the shape of the femoral region T of the wearer and move from places that are subjected to relatively high pressures to places that are subjected to relatively low pressures so that the entire pad 23 is enabled to evenly engage the femoral region T. In particular, even when the wearer squats, the femoral region T is enabled to contact the two support plates 22F and 22R over a large contact surface area, and the resulting even distribution of the contact pressure ensures a comfort to the wearer.

When there is a relative movement between the femoral support member 3 and femoral region T owing to the movement of the femoral region T, because the beads inside the pad 23 are in rolling contact with one another, the beads can move freely within the bag. Therefore, even when the pad 23 is in close contact with the femoral region T, the friction between the femoral region T and each of the support plates 22F and 22R is reduced by the rolling of the beads so that the relative movement between the femoral support member 3 and femoral region T can be favorably accommodated. Therefore, the movements of the femoral region T, in particular the flexing movement and twisting movement of the femoral region T, can be effected without obstruction.

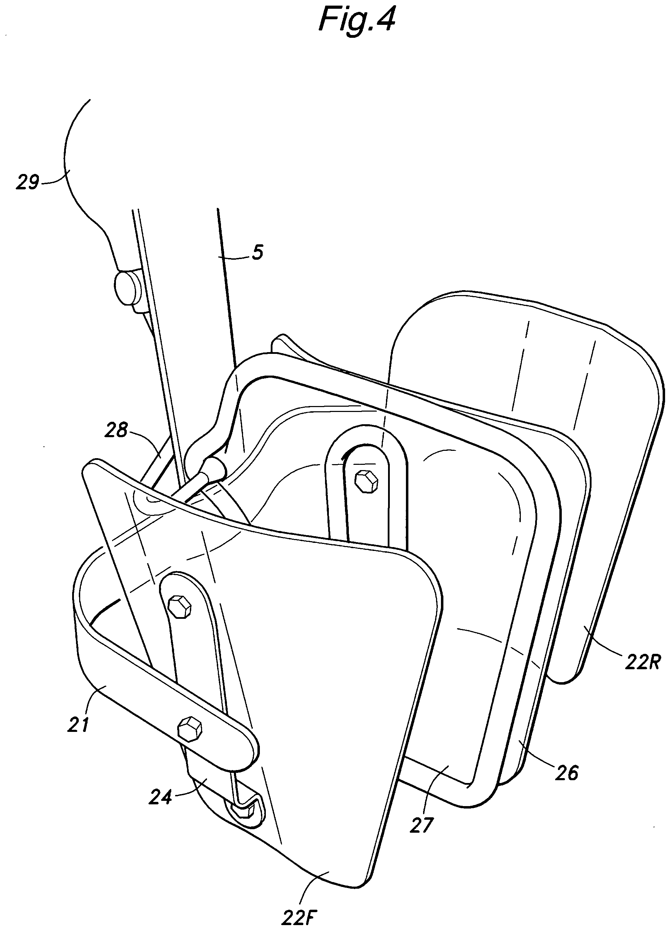

To the inner surface of the front support plate 22F is secured an air pad 27 by using a backup plate 26 having a substantially same shape as the front support plate 22F. This air pad 27 consists of a flattened blister made of soft elastomer material, and is provided with a part that is connected to a hand pump 29 via an air hose 28 as shown in Figure 4. Therefore, by pumping air into the air pad 27 by using the hand pump 29 after fastening the air belt 25, the femoral support member 3 can be even more firmly secured to the femoral region T. In this manner, the femoral support member 3 can be secured to the femoral region T with the benefit of the resiliency of the flexible connecting plate 24 and air pad 27.

The air cushioning action of the air pad 27 allows the air pad 27 to deform to such an extent as to accommodate deformation of the outer surface of the femoral region T owing to the swelling of the muscles such as when the user squats. Also, the securing force of the support member 3 can be readily adjusted by pumping air into and out of the air pad 27. These factors contribute to making the femoral support member 3 comfortable to wear. Because the femoral support member 3 can accommodate a large range of differences in the build of the user by using a simple structure, the femoral support member 3 can fit almost any person if it comes with a small number of different sizes such as S, M and L. Therefore, the femoral support member can be mass produced so that the manufacturing cost can be reduced as compared with the conventional femoral support member which is custom made for the build of each particular user.

### GLOSSARY

| | | | |
|---|---|---|---|
| 1 | walking assistance device | 3 | femoral support member |
| 4 | actuator | 5 | torque arm |
| 22F, 22R | support plate | 23 | pad |
| 24 | flexible connecting plate | 27 | air pad |
| T | femoral region | | |

## Claims

1. A walking assistance device (1), comprising a hip support member (2) adapted to be secured to a hip of a wearer, a power actuator (4) secured to the hip support member (2) and having a drive shaft, an assist force transmitting arm (5) extending radially from the drive shaft and a femoral support member (3) for securing a free end of the assist force transmitting arm to a femoral region (T) of the wearer, **characterized in that**:
the femoral support member (3) is adapted to engage the femoral region via an air pad (27) including a bag filled with air, the femoral support member (3) further comprises a stay (21) that supports the air pad (27) on a free end of the assist force transmitting arm (5) and is adapted to at least partly surround the femoral region (T) of the wearer, and **in that** the femoral support member (3) comprises a front support plate (22F) attached to the stay (21) so as to oppose a front face of the femoral region and a rear support plate (22R) attached to the stay (21) so as to oppose a rear face of the femoral region, the front support plate (22F) being adapted to engage a corresponding part of the femoral region via the air pad (27).

2. A walking assistance device (1) according to claim 1, wherein the assist force transmitting arm (5) consists of a member that is substantially rigid with respect to a rotational direction thereof.

3. A walking assistance device (1) according to claim 1 or 2, wherein each support plate (22F, 22R) is rotatably attached to the stay (21).

4. A walking assistance device (1) according to claim 1, 2 or 3, wherein each support plate (22F,22R) is attached to the stay (21) via a spring joint.

5. A walking assistance device (1) according to any preceding claim, wherein the air pad (27) is adapted to engage the femoral region (T) via a pad (23) including a bag and rolling granules filled in the bag.

6. A walking assistance device (1) according to any preceding claim, further comprising a pump (29) for supplementing air in the air pad (27).

## Patentansprüche

1. Gehhilfevorrichtung (1), umfassend ein Hüftstützbauteil (2), eingerichtet zum Befestigen an einer Hüfte eines Trägers, ein Kraftstellglieb (4), das an dem Hüftstützbauteil (2) befestigt ist und eine Antriebswelle aufweist, einen Übertragungsarm (5) für Unterstützungskraft, der sich radial von der Antriebswelle erstreckt, und ein femorales Stützbauteil (3) zum Befestigen eines freien Endes des Übertragungsarms für Unterstützungskraft an einer femoralen Region (T) des Trägers, **dadurch gekennzeichnet, dass**:
das femorale Stützbauteil (3) zum Eingriff mit der femoralen Region über ein Luftkissen (27), das einen mit Luft gefüllten Beutel enthält, eingerichtet ist, wobei das femorale Stützbauteil (3) weiter eine Strebe (21) umfasst, die das Luftkissen (27) an einem freien Ende des Übertragungsarms (5) für Unterstützungskraft stützt und eingerichtet ist, um mindestens teilweise die femorale Region (T) des Trägers zu umgeben, und dadurch, dass das femorale Stützbauteil (3) eine vordere Stützplatte (22F) umfasst, die an der Strebe (21) angebracht ist, sodass sich eine vordere Fläche der femoralen Region und eine hintere Stützplatte (22R), angebracht an der Strebe (21), gegenüberliegen, sodass eine hintere Fläche der femoralen Region gegenüberliegt, wobei die vordere Stützplatte (22F) zum Eingriff mit einem entsprechenden Teil der femoralen Region über das Luftkissen (27) eingerichtet ist.

2. Gehhilfevorrichtung (1) nach Anspruch 1, wobei der Übertragungsarm (5) für Unterstützungskraft aus einem Bauteil besteht, das hinsichtlich einer Rotationsrichtung davon im Wesentlichen starr ist.

3. Gehhilfevorrichtung (1) nach Anspruch 1 oder 2, wobei jede Stützplatte (22F, 22R) drehbar an der Strebe (21) angebracht ist.

4. Gehhilfevorrichtung (1) nach Anspruch 1, 2 oder 3, wobei jede Stützplatte (22F, 22R) an der Strebe (21) über ein Federgelenk angebracht ist.

5. Gehhilfevorrichtung (1) nach einem der vorstehenden Ansprüche, wobei das Luftkissen (27) zum Eingriff mit der femoralen Region (T) über ein Kissen (23) eingerichtet ist, das einen Beutel und Rollgranulat beinhaltet, das in den Beutel gefüllt ist.

6. Gehhilfevorrichtung (1) nach einem der vorstehenden Ansprüche, weiter umfassend eine Pumpe (29) zum Ergänzen von Luft in dem Luftkissen (27).

## Revendications

1. Dispositif d'assistance à la marche (1), comprenant un élément de support de hanche (2) qui est à même d'être fixé à la hanche d'un utilisateur, un actionneur mécanique (4) fixé à l'élément de support de hanche (2) et ayant un arbre d'entraînement, un bras de transmission de force d'assistance (5) s'étendant radialement de l'arbre d'entraînement et un élément de support fémoral (3) pour fixer une extrémité libre du bras de transmission de force d'assistance à une région fémorale (T) de l'utilisateur, **caractérisé en ce que** :
l'élément de support fémoral (3) est à même de s'engager sur la région fémorale via un coussin d'air (27) comprenant un sac rempli d'air, l'élément de support fémoral (3) comprend en outre un appui (21) qui supporte le coussin d'air (27) sur une extrémité libre du bras de transmission de force d'assistance (5) et est à même d'entourer au moins en partie la région fémorale (T) de l'utilisateur, et **en ce que** l'élément de support fémoral (3) comprend une plaque de support avant (22F) fixée à l'appui (21) de manière à être en regard d'une face avant de la région fémorale et une plaque de support arrière (22R) fixée à l'appui (21) de manière à être en regard d'une face arrière de la région fémorale, la plaque de support avant (22F) étant à même de s'engager sur une partie correspondante de la région fémorale via le coussin d'air (27).

2. Dispositif d'assistance à la marche (1) selon la revendication 1, dans lequel le bras de transmission de force d'assistance (5) est constitué d'un élément qui est sensiblement rigide par rapport à une direction de rotation de celui-ci.

3. Dispositif d'assistance à la marche (1) selon la revendication 1 ou 2, dans lequel chaque plaque de support (22F, 22R) est fixée de manière rotative à l'appui (21).

4. Dispositif d'assistance à la marche (1) selon la revendication 1, 2 ou 3, dans lequel chaque plaque de support (22F, 22R) est fixée à l'appui (21) via un joint à ressort.

5. Dispositif d'assistance à la marche (1) selon l'une quelconque des revendications précédentes, dans lequel le coussin d'air (27) est à même de s'engager sur la région fémorale (T) via un coussin (23) comprenant un sac et des granules roulants dont le sac est rempli.

6. Dispositif d'assistance à la marche (1) selon l'une quelconque des revendications précédentes, comprenant en outre une pompe (29) pour ajouter de l'air dans le coussin d'air (27).
